(19) Europäisches Patentamt
European Patent Office
Office européen des brevets

(11) Veröffentlichungsnummer : **0 007 076**
**B1**

(12) **EUROPÄISCHE PATENTSCHRIFT**

(45) Veröffentlichungstag der Patentschrift : **15.04.81**

(51) Int. Cl.³ : **C 07 C 13/16**, A 61 K 7/46, C 07 C 33/05, C 09 D 7/12, C 11 D 3/50

(21) Anmeldenummer : **79102304.7**

(22) Anmeldetag : **06.07.79**

(54) **Dihydrobisabolene und Dihydrobisabolol, deren Herstellung und Verwendung als Duftstoffe.**

(30) Priorität : **14.07.78 DE 2830971**

(43) Veröffentlichungstag der Anmeldung : **23.01.80 (Patentblatt 80/02)**

(45) Bekanntmachung des Hinweises auf die Patenterteilung : **15.04.81 Patentblatt 81/15**

(84) Benannte Vertragsstaaten : **CH DE FR GB NL**

(56) Entgegenhaltungen : **Keine**
**US - E - 29 649**

**CHEMICAL ABSTRACTS, Band 77, Nr. 13, 25. September 1972 Columbus, Ohio, U.S.A. N.H. ANDERSEN et al. : « Chemical stimulation of the biogenesis of cedrene », Seiten 482 und 483, Abstract Nr. 88 697 F.**

**CHEMICAL ABSTRACTS, Band 84, Nr. 19, 10. Mai 1976 Columbus, Ohio, U.S.A. N.H. ANDERSEN « From nerolidol to cedrene and vetiver tricyclics. Chemical realization of the stepwise processes », Seite 520, Abstract Nr. 135 845 J.**

(73) Patentinhaber : **BASF Aktiengesellschaft**
**Carl-Bosch-Strasse 38**
**D-6700 Ludwigshafen (DE)**

(72) Erfinder : **Hoffmann, Werner, Dr. Chem.**
**Ringstrasse 11c**
**D-6701 Neuhofen (DE)**
Erfinder : **Thoemel, Frank, Dr. Chem.**
**Leberstrasse 17**
**D-6940 Weinheim (DE)**

**EP 0 007 076 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Dihydrobisabolene und Dihydrobisabolol, deren Herstellung und Verwendung als Duftstoffe

Die Erfindung betrifft Dihydrobisabolene (1) und Dihydrobisabolol (2)

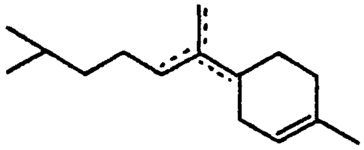

(1)

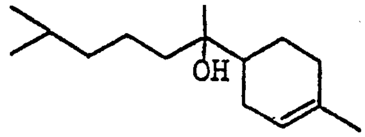

(2)

d.h. Verbindungen der allgemeinen Formel I

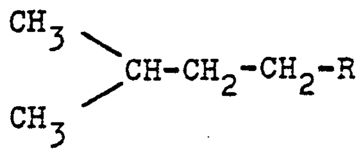

I,

in der R für

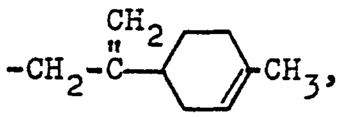

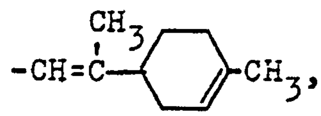

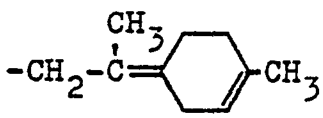

oder

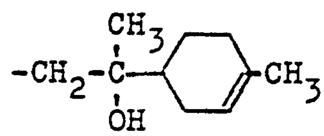

steht, deren Herstellung und Verwendung als Duftstoffkomponente in Duftstoffkompositionen, die in kosmetischen Zubereitungen aller Art sowie in Wasch- und Reinigungsmitteln oder als Duftverbesserer in Leimen, Anstrichmitteln und ähnlichen Erzeugnissen.

Bisabolen (3) und Bisabolol (4)

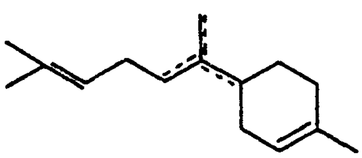

(3)

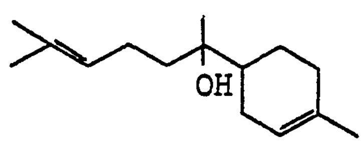

(4)

sind bekannte Riechstoffe mit Blumen- bzw. Holzduftnoten, die sich steigender Beliebtheit erfreuen.

Es wurde nun gefunden, daß die Dihydrobisabolene (1) und Dihydrobisabolol (2) ebenfalls sehr gute Riechstoffe sind, die sich durch angenehme Blumen- und Grünnoten auszeichnen, sich aber durch interessante Duftnuancen von diesen unterscheiden und außerdem eine wesentlich höhere Stabilität und auch höhere thermische Stabilität gegenüber Luftsauerstoff aufweisen. Einen Vergleich der thermischen Stabilität von Dihydrobisabolol mit der von Bisabolol zeigt Beispiel 2.

Während die Peroxidzahl bei der Behandlung von Bisabolol (4) mit Luft ständig ansteigt und dabei eine Gelbfärbung eintritt, bleibt sie beim Dihydrobisabolol (2) unter gleichen Bedingungen praktisch konstant und das Produkt farblos, d.h. Dihydrobisabolol erleidet bei seiner Anwendung keine Veränderung in Geruch und Farbe.

Es wurde ferner gefunden, daß man die neuen Dihydrobisabolene und das Dihydrobisabolol der Formel I sehr vorteilhaft herstellen kann, indem man das technisch gut zugängliche Dihydronerolidol durch Einwirken einer starken organischen Säure oder einer schwachen organischen Säure in Gegenwart von 0,001 bis 0,1 Mol Schwefelsäure oder einer Lewis-Säure pro Mol organische Säure cyclisiert, ggf. das dabei gebildete, im wesentlichen aus Dihydrofarnesen, Dihydrobisabolen, einem Dihydrobisabololester und einem Dihydrofarnesolester bestehende Gemisch alkalisch hydrolysiert oder basenkatalysiert umestert und dann wie üblich aufarbeitet.

Das Dihydronerolidol (3,7,11'-Trimethyl-dodeca-1,6 dien-3-ol) läßt sich nach bekannten Methoden

beispielsweise aus Methyl-heptanon durch Äthinylierung mit anschließender Partialhydrierung, Carroll-Reaktion mittels Acetessigester und erneute Äthinylierung mit anschließender Partialhydrierung relativ gut herstellen.

Als organische Säure für die Cyclisierung eignen sich besonders Ameisensäure, Essigsäure und Propionsäure, sowie Toluolsulfonsäure in Toluol. Die Ameisensäure verwendet man im allgemeinen in Form einer konzentrierten wäßrigen Säure, d.h. einer etwa 50- bis 99,9 %-igen wäßrigen HCOOH-Lösung, vorzugsweise einer 80- bis 99 %-igen HCOOH-Lösung oder wasserfreie HCOOH. Bei Verwendung von Essigsäure oder Propionsäure empfiehlt sich der Zusatz einer stärkeren Säure wie Schwefelsäure oder einer Lewis-Säure wie Bortrifluoridätherat, Zinkchlorid, Aluminiumchlorid, Eisen(III)-chlorid und Zinn(IV)-chlorid. Der Zusatz der Schwefelsäure bzw.der Lewissäure in der Essigsäure oder Propionsäure beträgt im allgemeinen etwa 0,001 bis 0,1 Mol pro Mol Essigsäure oder Propionsäure.

Die organische Säure verwendet man zur Cyclisierung im allgemeinen in Mengen von 1 bis 20 Mol, vorzugsweise 3 bis 15 Mol pro Mol Dihydronerolidol.

Die Cyclisierung führt man im allgemeinen bei Temperaturen von —20 bis 100 °C, vorzugsweise 0 bis 60 °C, insbesondere 10 bis 40 °C durch. Die Reaktionsdauer beträgt je nach der zur Cyclisierung verwendeten Säure und je nach der Reaktions-temperatur 1 bis 300 Minuten, vorzugsweise zwischen 10 und 120 Minuten.

Zur Durchführung der Cyclisierung erwärmt man im allgemeinen die Säure bzw. das Säuregemisch auf die Reaktionstemperatur und trägt anschließend unter Rühren und schwachem Kühlen das Dihydronerolidol ein.

Bei der beschriebenen Cyclierungsreaktion bilden sich neben dem Gemisch der isomeren Dihydrobisabolene das Dihydrofarnesen sowie die Ester aus der zur Cyclisierung verwendeten organischen Säure und Dihydrobisabolol sowie die entsprechenden Ester von Dihydrofarnesol. Das Verhältnis der sich bildenden Produkte ist stark von der Art der zur Cyclisierung verwendeten Säure, von der Temperatur sowie von der Verweilzeit abhängig. Höhere Temperaturen und längere Verweilzeiten begünstigen die Olefinbildung.

Aus dem bei der Cyclisierung gebildeten Produktgemisch kann man die einzelnen Verfahrensprodukte durch fraktionierte Destillation isolieren. Mit besonderem Vorteil aber unterwirft man das anfallende Reaktionsgemisch in an sich bekannter Weise einer alkalischen Hydrolyse oder einer basenkatalysiertem Umesterung, wodurch aus den Estern die Alkohole als solche freigesetzt werden und arbeitet die hierbei anfallende abtrennbare organische Phase destillativ auf.

Zur alkalischen Hydrolyse verwendet man im allgemeinen etwa 1,1 bis 2 Mol einer etwa 10- bis 20 %-igen wäßrigen NaOH-Lösung, berechnet auf vorhandene Ester. Das anfallende Reaktionsgemisch wird zu einer NaOH-Lösung zugegeben und bei 30 bis 60 °C so lange gerührt bis mit Hilfe der Gaschromatographie oder Dünnschichtchromatographie keine Ester mehr nachweisbar sind.

Zur basenkatalysierten Umesterung verwendet man im allgemeinen pro Mol Ester 2 bis 5 Mol Methanol sowie katalytische Mengen an $NaOCH_3$ (0,001 bis 0,01 Mol pro Mol Ester). Man erhitzt das Reaktionsgemisch mit der $CH_3OH$-$NaOCH_3$-Lösung auf 50 bis 100 °C, wobei man den gebildeten Ameisensäuremethylester oder Essigsäuremethylester über eine Kolonne abdestillieren läßt. Das Ende der Umesterung ist hierbei an dem Aufhören der Methylesterbildung zu erkennen.

Die neuen Dihydrobisabolene und das Dihydrobisabolol sind wertvolle Duftstoffe für kosmetische Zubereitungen aller Art sowie für Wasch- und Reinigungsmittel bzw. wertvolle Duftverbesserer für technische Produkte wie Leime, Anstrichmittel und ähnliche Erzeugnisse. Sie können entweder in reiner Form oder in Mischung mit anderen Duftstoffen verwendet werden. Die Mengen dieser Duftstoffe entsprechen denen von bekannten Geruchsträgern. Sie können technisch auf relativ einfache Weise vollsynthetisch hergestellt werden.

Beispiel 1

Herstellung von Dihydrobisabolen und Dihydrobisabolol

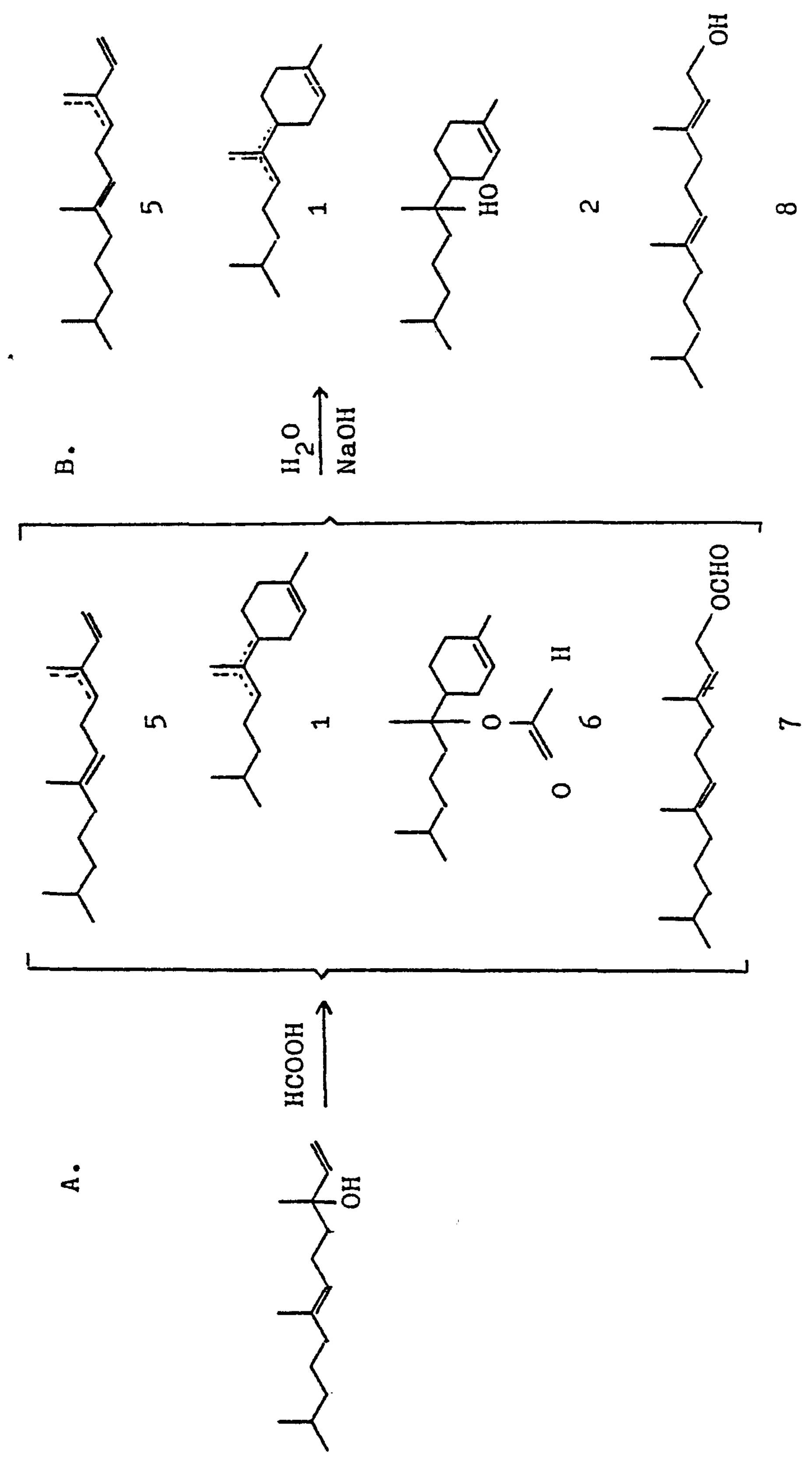

A. Cyclisierung

2 530 g (50 Mol) 90 %-ige Ameisensäure wurde auf 40 °C erwärmt und unter Rühren und schwacher Kühlung mit einem Wasserbad mit 1 120 g (5 Mol) 3,7,11-Trimethyl-dodeca-1,6-dien-3-ol (Dihydronerolidol) versetzt. Zulauf und Kühlung wurden so abgestimmt, daß die Reaktionstemperatur 40 °C betrug. Der Reaktionsverlauf wurde durch gaschromatographische Analyse verfolgt. Nachdem eine Stunde nach Zulaufende kein Ausgangsmaterial mehr nachweisbar war, wurde aufgearbeitet. Dazu wurde die organische Phase abgetrennt und in der Säurephase unter titrimetrischer Kontrolle die verbrauchte Ameisensäure ergänzt. Mit der ergänzten Säurephase wurden erneut 1 120 g Dihydronerolidol auf die oben beschriebene Weise umgesetzt.

Insgesamt wurde die Ameisensäure dreimal auf diese Weise verwendet, wobei insgesamt 3 503 g organische Phase anfielen. Nach gaschromatographischer Analyse bestand das erhaltene Produkt zu ca. 20 % aus den Kohlenwasserstoffen 1 und 5 und zu ca. 80 % aus den Formiaten 6 und 7.

B. Hydrolytische Aufarbeitung des Cyclisierungsansatzes

In 2 550 ml Wasser wurden 645 g Natriumhydroxid gelöst und unter Rühren bei 50 °C mit 3 500 g der gemäß 1A erhaltenen organischen Phase versetzt. Nach Zulaufende wurden stündlich Proben entnommen und der Reaktionsverlauf gaschromatographisch verfolgt. Nach 5 Stunden waren keine Formiate mehr nachweisbar. Die organische Phase wurde abgetrennt, mit Wasser neutral gewaschen und das restliche Wasser durch Trocknen bei 50 °C und 20 Torr entfernt. Es verblieben 2 975 g eines Produktgemisches, das nach gaschromatographischer Analyse folgende Zusammensetzung hatte : ca. 38 % Dihydrobisabolol (2), 17 % Dihydrobisabolen (1), 38 % Dihydrofarnesol (8) und 6 % Dihydrofarnesen (5). Durch fraktionierte Destillation über eine 1,5 m-Kolonne wurden daraus isoliert :

*Dihydrofarnesen (5)* = E,Z-3,7,11-Trimethyl-dodeca-1,3,5-trien und E,Z-7,11-Dimethyl-3-methylen-dodeca-1,5-dien

169 g (Ausbeute 5,5 %)

Kp. 69 bis 71 °C/0,1 mbar ; $n_D^{25} = 1,4610$

*Dihydrobisabolen (1)* = Gemisch aus 1-Methyl-4-(1,5-dimethyl-1-hexenyl)-1-cylohexen (3a), 1-Methyl-4-(5-methyl-1-methylen-hexanyl)-1-cyclohexen (3b) und 1-Methyl-4-(1,5-dimethyl-hexyliden)-1-cyclohexen (3c)

478 g (Ausbeute 15,5 %)

Kp. 76 bis 77 °C/0,1 mbar ; $n_D^{25} = 1,4740$

Geruch : grün, heuartig, pilzig

Nach $C_{13}$-MMR-Spektrum sind die Isomeren 3 wie folgt verteilt :

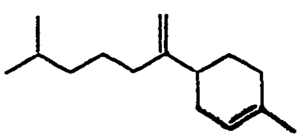

15 % (3b)

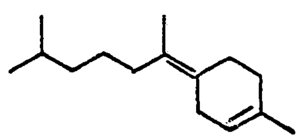

25 % (3c)

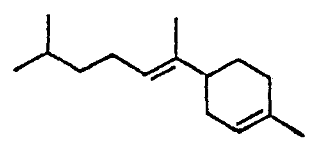

60 % (3a)

*Dihydrobisabolol (2)* 1-Methyl-4-(1,5-dimethyl-1-hydroxy-hexanyl)-1-cyclohexen

1 090 g (Ausbeute 32,5 %)

Kp. 89 bis 93 °C/0,01 mbar ; $n_D^{25} = 1,4766$

Geruch : mild, blumig, orchideenartig

*Dihydrofarnesol (8)* E,Z-3,7,11-Trimethyl-dodeca-2,5-dien-1-ol

1 090 g (Ausbeute 32,5 %)

Kp. 95 bis 98 °C/0,005 mbar ; $n_D^{25} = 1,4738$

## Beispiel 2 (Vergleichsbeispiel)

Vergleich der Luftempfindlichkeit von Dihydrobisabolol (2) mit derjenigen von Bisabolol

100 g Dihydrobisabolol und 100 g Bisabolol wurden jeweils in einen 250 ml-Rührkolben gegeben und durch die Flüssigkeit unter Rühren bei 50 °C ein Luftstrom (ca. 10 1/h) hindurchgeleitet. Nach den in

der Tabelle angegebenen Zeiten wurden Proben entnommen und der Peroxid-Sauerstoff nach der Eisessig-KJ-Methode bestimmt.

| Zeit (h) | Dihydrobisabolol Peroxid-Sauerstoff (%) | Bisabolol Peroxid-Sauerstoff (%) |
|---|---|---|
| 0 | 0,064 | 0,039 |
| 1 | 0,021 | 0,063 |
| 2 | 0,021 | 0,066 |
| 3 | 0,023 | 0,075 |
| 4 | 0,019 | 0,092 |
| 5 | 0,025 | 0,12 |
| 12 | 0,035 | 0,33 |

## Beispiel 3

Cyclisierung mit Essigsäure/konz. Schwefelsäure

A. Cyclisierung

Zu einem Gemisch von 600 g (10 Mol) Essigsäure und 19,6 g (0,2 Mol) konz. Schwefelsäure tropfte man bei 20 °C in 15 Minuten 224 g (1 Mol) Dihydronerolidol.

Das Reaktionsgemisch wurde bei 20 °C gerührt und der Reaktionsverlauf durch dünnschichtchromatographische Analyse (Fertigplatten Kieselgel, Merck ; Laufmittel Cyclohexan/Essigsäure-Alkylester 3:1 ; Sprühmittel gesättigte wäßrige Kaliumpermanganatlösung, mit etwas Schwefelsäure versetzt) verfolgt. Nach 90 Minuten hatte sich das Dihydronerolidol vollständig umgesetzt. Die organische Phase wurde von der Säurephase abgetrennt und mit Wasser gewaschen. Den Cyclisierungsansatz arbeitete man wie folgt direkt hydrolytisch auf :

B. Aufarbeitung

In 510 g Wasser wurden 130 g Natriumhydroxid gelöst und unter Rühren bei 50 °C mit 209 g der organischen Phase versetzt. Man ließ 1 Stunde bei 50 °C nachrühren, trennte die organische Phase ab und wusch sie neutral, trocknete mit wasserfreiem Natriumsulfat und destillierte an einer Destillationsbrücke : Kp 89 bis 160 °C/0,1 mbar ; Ausbeute 179 g, Destillationsrückstand 16 g. Das Destillationsprodukt enthielt laut gaschromatographischer Analyse 89 % Dihydrobisabolen (1), 8 % Dihydrobisabolol (2) und 3 % unbekannte Produkte.

## Beispiel 4

600 g (10 Mol) Essigsäure, 2 g (0,02 Mol) konz. Schwefelsäure und 224 g (1 Mol) Dihydronerolidol wurden analog Beispiel 3A bei 20 °C umgesetzt. Nach 12 Stunden ließ sich dünnschichtchromatographisch kein Ausgangsmaterial mehr nachweisen. Die Aufarbeitung des Reaktionsproduktes erfolgte durch Zusatz von 500 g Wasser, Abtrennen der organischen Phase und Neutralwaschen mit Wasser. Die organische Phase enthielt 48,8 % Dihydrobisabolylacetat im Gemisch mit Dihydrofarnesylacetat, der Rest bestand aus unbekannten Produkten. Die Hydrolyse der organischen Phase (226 g) wurde analog Beispiel 3B durchgeführt. Dazu wurden 130 g Natriumhydroxid in 510 g Wasser gelöst, mit den 226 g der organischen Phase bei 50 °C versetzt. Reaktionszeit 1 Stunde. Das organische Produkt wurde abgetrennt und über eine Destillationsbrücke destilliert : Kp 78 bis 147 °C/0,1 mbar, Ausbeute 180 g, Destillationsrückstand 20 g. Das Destillationsprodukt enthielt laut gaschromatographischer Analyse 45,2 %-Dihydrobisabolen (1), 40,2 % Dihydrobisabolol (2), 8,8 % Dihydrofarnesol (8) und 5,8 % unbekannte Produkte.

## Beispiel 5

Cyclisierung in Toluol mit p-Toluol-sulfonsäure

Eine Lösung von 34,4 g (0,2 Mol) p-Toluolsulfonsäure in 600 g Toluol wurde auf 40 °C erwärmt und innerhalb von 15 Minuten mit 224 g (1 Mol) Dihydronerolidol versetzt. Der Reaktionsverlauf wurde durch dünnschichtchromatographische Analyse (vgl. Beispiel 3) verfolgt. Nach 1 Stunde hatte sich alles Ausgangsmaterial umgesetzt. Das Reaktionsprodukt wurde mit Wasser neutral gewaschen und das Toluol unter vermindertem Druck abdestilliert. Der Rückstand (210 g) wurde analog Beispiel 1B

hydrolysiert. Dazu wurden 130 g Natriumhydroxid in 510 g Wasser gelöst und bei 50 °C mit dem erhaltenen Rückstand versetzt. Reaktionszeit 2 Stunden. Die organische Phase wurde abgetrennt und über eine Destillationsbrücke abdestilliert : Kp 67 bis 105 °C/0,1 mbar, Ausbeute 155 g, Destillationsrückstand 36 g. Das Destillationsprodukt enthielt laut gaschromatographischer Analyse 91,2 % Dihydrobisabolen (1), 8 % Dihydrobisabolol (2) und 0,8 % unbekannte Produkte.

**Ansprüche**

1. Dihydrobisabolene und Dihydrobisabolol der allgemeinen Formel I

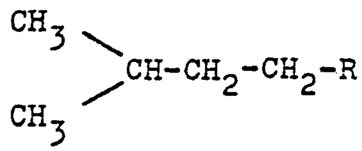

I,

in der R für

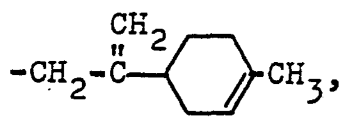

,

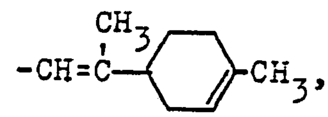

,

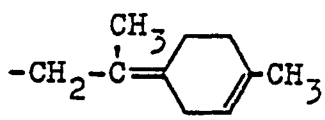

oder

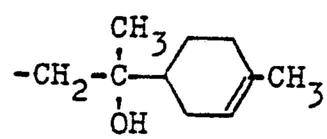

steht.

2. 1-Methyl-4-(1,5-dimethyl-1-hydroxyhexanyl)-1-cyclohexen

3. Gemisch aus 1-Methyl-4-[1,5-dimethyl-1-hexenyl]-1-cyclohexen ; 1-Methyl-4-[5-methyl-1-methylen-hexanyl]-1-cyclohexen und 1-Methyl-4-[1,5-dimethyl-hexyliden]-1-cyclohexen.

4. verfahren zur Herstellung von Dihydrobisabolenen und Dihydrobisabolol der allgemeinen Formel I gemäß Anspruch 1, *dadurch gekennzeichnet,* daß man Dihydronerolidol durch Einwirken einer starken organischen Säure oder einer schwachen organischen Säure in Gegenwart von 0,001 bis 0,1 Mol Schwefelsäure oder einer Lewissäure pro Mol organischer Säure cyclisiert, das dabei gebildete, im wesentlichen aus Dihydrofarnesen, Dihydrobisabolen, einem Dihydrobisabololester und einem Dihydrofarnesolester bestehende Gemisch alkalisch hydrolysiert oder basenkatalysiert umestert und dann wie üblich aufarbeitet.

5. Verwendung der Dihydrobisabolene und des Dihydrobisabolols der allgemeinen Formel I gemäß Anspruch 1 als Duftstoffkomponente für Duftstoffkompositionen, für kosmetische Zubereitungen sowie für Wasch- und Reinigungsmittel und für Duftverbesserer in Leimen und Anstrichmitteln.

**Claims**

1. Dihydrobisabolenes and dihydrobisabolol of the general formula I

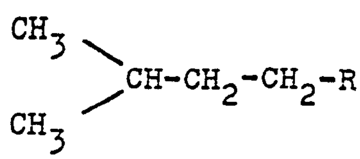

I,

where R is

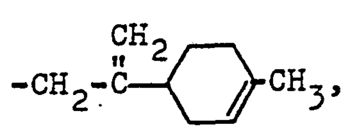

,

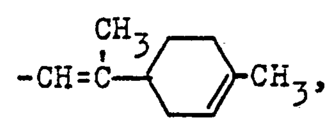

,

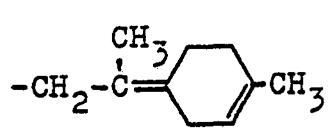

or

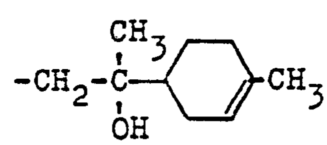

2. 1-methyl-4-(1,5-dimethyl-1-hydroxy-hexanyl)-1-cyclohexene.

3. A mixture of 1-methyl-4-[1,5-dimethyl-1-hexenyl]-1-cyclohexene, 1-methyl-4-[5-methyl-1-methylene-hexanyl]-1-cyclohexene and 1-methyl-4-[1,5-dimethyl-hexylidene]-1-cyclohexene.

4. A process for the production of dihydrobisabolenes and dihydrobisabolol of the general formula I, as claimed in claim 1, *characterized in that* dihydronerolidol is cyclized by the action of a strong organic acid or a weak organic acid in the presence of from 0.001 to 0.1 mole of sulphuric acid or a Lewis acid per mole of organic acid, the resulting mixture consisting essentially of dihydrofarnesene, dihydrobisabolene, a dihydrobisabolol ester and a dihydrofarnesol ester is subjected to alkaline hydrolysis or reesterified in the presence of a basic catalyst, and then worked up as usual.

5. Use of a dihydrobisabolene and dihydrobisabolol of the general formula I, as claimed in claim 1, as a component of odorant compositions for cosmetic preparations and for detergents and cleaning products and for improving the odour of glues and surface coating compositions.

**Revendications**

1. Dihydro-bis-abolènes et dihydro-bis-abolol de formule générale I

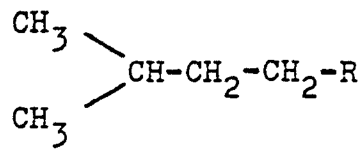 I,

dans laquelle R représente

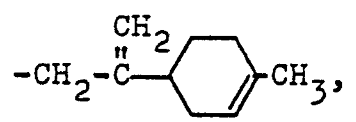 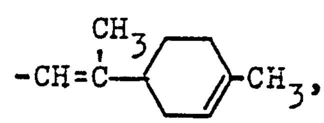

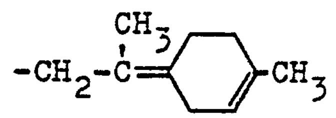 ou 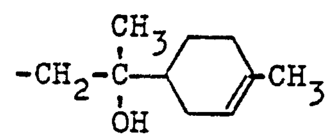

2. 1-méthyl-4-(1,5-diméthyl-1-hydroxy-hexanyl)-1-cyclohexène.

3. Mélange de 1-méthyl-4-[1,5-diméthyl-1-hexényl]-1-cyclohexène ; 1-méthyl-4-[5-méthyl-1-méthylène-hexanyl]-1-cyclohexène et 1-méthyl-4-[1,5-diméthyl-hexylidène]-1-cyclohexène.

4. Procédé de préparation des dihydro-bis-abolènes et du dihydro-bis-abolol de formule générale I selon la revendication 1, caractérisé en ce que l'on soumet le dihydronérolidol à cyclisation sous l'action d'un acide organique fort ou d'un acide organique faible en présence de 0,001 à 0,1 mole d'acide sulfurique ou d'un acide de Lewis par mole d'acide organique, on soumet le mélange obtenu, consistant essentiellement en dihydrofarnésène, dihydro-bis-abolène, un ester de dihydro-bis-abolol et un ester de dihydrofarnésol à hydrolyse alcaline ou à transestérification catalysée par une base et on termine de la manière habituelle.

5. Utilisation des dihydro-bis-abolènes et du dihydro-bis-abolol de formule générale I selon la revendication 1 en tant que composants odorants pour compositions de parfums pour produits cosmétiques, pour produits de lavage et de nettoyage et pour correcteurs d'odeurs dans des colles et produits de revêtements.